# EUROPEAN PATENT APPLICATION

(11) **EP 1 199 076 A2**
(43) Date of publication of application: **24.04.2002**
(21) Application number: 01123298.0
(22) Date of filing: 05.10.2001
(51) Int. Cl.: A61K 31/353, A61P 7/00, A61P 9/10, A61P 9/00

(54) **Medicament containing tocotrienol as active component**

(30) Priority: 06.10.2000 JP 2000307252
(71) Applicant: FUJI CHEMICAL INDUSTRY CO LTD, Nakaniikawa-gun, Toyama (JP)
(72) Inventor: Terao, Junji, Dep. of Nutrition,School of Medicine, Tokushima-shi, Tokushima-ken (JP); Ikushima, Heiji, Fuji Chemical Industry Co. Ltd., Nakaniikawa-gun, Toyama (JP)
(74) Representative: Albrecht, Thomas, Dr.

(57) **Abstract**

The present invention provides an agent for ischemic disease, for improving deformability of erythrocyte membrane and for improving peripheral circulation, which comprises a tocotrienol compound as an active component.

## Description

### BACKGROUND OF THE INVENTION

This invention relates to an agent for ischemic disease or the improvement in the deformability of erythrocyte membrane or peripheral circulation, which comprises a tocotrienol compound as an active component.

Recently, it has been noted the relation between the trouble caused by oxidation induced by free radicals and coronary heart disease, cancer, aging or the like, particularly oxidation of lipids biomembrane in relation to deterioration of membrane structure and troubles in enzymatic function. It is possible that antioxidant reduces risk of suffering from such a degeneration disease by inhibiting the oxidation troubles induced by free radicals, and there is a report about inhibition against oxidation of lipids by natural and synthetic antioxidants.

Various investigations have been achieved about utilization of tocotrienol, which is a natural antioxidant, for prevention and treatment of various diseases. For example, (1) lowering of the level of cholesterol in blood (American journal of Medicine, 62, 707 714 (1977), (2) use as anti-inflammatory, immunomodulator, or for the treatment or prevention of hypercholesterolemia, thrombosis, oxidative or atherogenic symptom, decrease of Lp(a) level in blood, fever, edema, diabetes mellitus, pain, septic shock, chronic fatigue syndrome, functio lacsa and the like (Japanese Patent KOHYO 7-504887), (3) therapeutic agent of α-tocotrienol for hypercholesterolemia (J. Biol. Chem. 261, 10, 544-550 (1986)), (4) use for hypercholesterolemia, hyperlipemia and obstructive thrombosis (EP 421, 419A), (5) therapeutic agent for oxygen disorder (Japanese Patent KOKAI 58-96021), (6) antitumor agent (Japanese Patent KOKAI 61-210030), (7) hair-growing agent (Japanese Patent KOKAI 8-12532), (8) deodorant and compositions for oral cavity (Japanese Patent KOKAI 8-92050), (9) epithem for skin (Japanese Patent KOKAI 8-92062), (10) patch (Japanese Patent KOKAI 9-208460), (11) immunofunction improving agent (Japanese Patent KOKAI 11-49767) and so on.

However, no report has been made as to any action of a tocotrienol compound on the improvement in the deformability of erythrocyte membrane. For example, the above Japanese Patent KOKAI 58-96021 discloses that tocotrienol compounds and esters thereof are incorporated into biomembrane through tocols (α-, β-, γ- and δ-tocopherols), α-tocopherols exhibits to inhibit hemolysis by dialuric acid even in an amount of one third of α-tocotrienol, and α-tocopherol can be incorporated thereinto much easier than tocols (tocopherols). However, there is no suggestion about the improvement in the deformability of erythrocyte membrane.

Japanese Patent KOKAI 2000-169369 discloses that tocotrienols are effective in the treatment of coronary heart disease and thrombosis caused by high cholesterol level or the like, but there is no description about the improvement in the deformability of erythrocyte membrane.

Although Japanese Patent KOKAI 9-208460 discloses tocotrienols blended with a patch to accelerate bloodstream to exhibit treatment effects on diseases in orthopedics region, such as stiffness of shoulder, muscular pain and the like, the evaluation was achieved by measuring human blood stream by a laser Doppler rheometer after attaching the patch. There is no description about the improvement in the deformability of erythrocyte membrane.

### SUMMARY OF THE INVENTION

An object of the invention is to provide a novel agent for ischemic disease, for improving deformability of erythrocyte membrane or for improving peripheral circulation.

As a result of researching the novel agent, the inventors found that tocotrienol compounds exhibit excellent action on the improvement in the deformability of erythrocyte membrane.

The present invention has been made based on the finding, and provides
an agent for ischemic disease which comprises a tocotrienol compound as an active component,
an agent for improving deformability of erythrocyte membrane which comprises a tocotrienol compound as an active component,
an agent for improving peripheral circulation which comprises a tocotrienol compound as an active component.

### BRIEF DESCRIPTION OF THE DRAWINGS

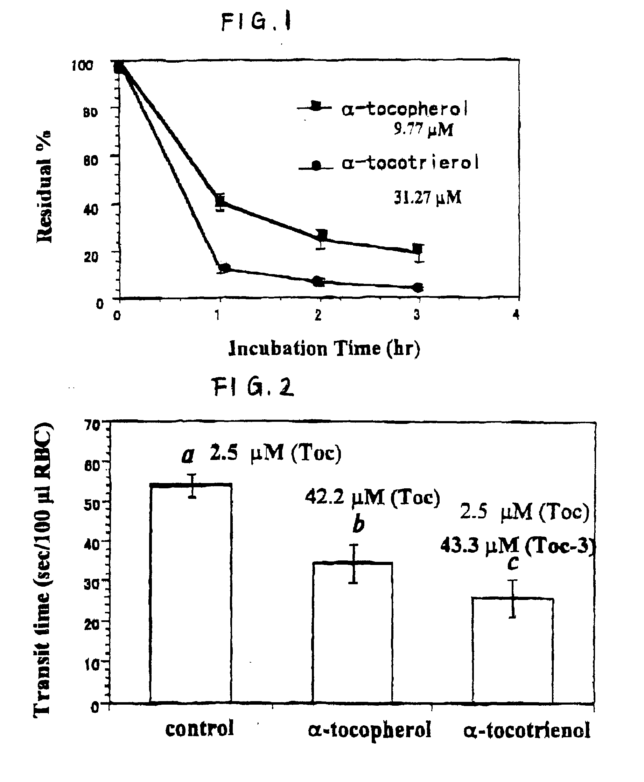

Figure 1 is a graph showing reactivity of α-tocotrienol in human erythrocyte membrane with radicals. The numbers on the ordinate indicate the rate of residues (%) of tocotrienol ( 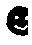 ) and tocopherol (■), and the numbers on the abscissa indicate incubation time.
Figure 2 is a graph showing influence of α-tocotrienol on the deformability of human erythrocyte membrane. In the figure, a is control, b is tocopherol (Toc), c is tocotrienol (Toc-3), respectively. The numbers on the ordinate indicate passing time [µ1 erytthrocytes (RBC)].
Figure 3 is a schematic illustration of the measuring device of the deformability of erythrocyte [Microchannel Array Flow Analyzer (MC-FAN)]. In the figure, M is microscope, GP is glass plate, SS is silicon base plate, D is the length of slit, W is the width of slit, and L and 1 are flow pathes, respectively. The silicon base plate constituting micro-flow pathes is in contact with the glass plate with pressure. Both plates have been provided with optical polishing.

### DETAILED DESCRIPTION OF THE INVENTION

The tocotrienol compound applicable to the invention includes tocotrienol, α-tocotrienol, β-tocotrienol, γ-tocotrienol, δ-tocotrienol, nicotinate esters of the above tocotrienol isomers, and the like. The tocotrienol compound may be any of d-, 1 or d1-isomer, and further may be a mixture of two or more of the above tocotrienol compounds.

The tocotrienol compound can be produced by known method, such as pressing a natural material, extraction from a natural material, synthesis of the like. The tocotrienol compound may be purified, for example, by column chromatography.

The agent in the invention can be used for a pharmaceutical composition for ischemic disease, for the improvement in the deformability of erythrocyte membrane, or for the improvement in peripheral circulation in various forms which are administrated orally or parenterally.

A suitable dose varies depending on age, sexuality, body weight and susceptibility of patient, method of administration, phase of administration, sensitivity, recipe, form of drug and the like, and typically, is about 0.1 to 5,000 mg, preferably 0.5 to 3,000 mg, more preferably 1 to 2,000 mg per adult per one day.

The pharmaceutical composition can be prepared into various forms, such as tablets, sublingual tablets, pills, suppository, triturate, powder, parvules, granules, capsules, microcapsules, injection, emulsion, patches and the like. For example, tablets can be produced by mixing pharmacologically acceptable carrier homogeneously with the composition, and then tabletting the mixture. Triturate, powder and granules can be produced by rendering the composition into solution or suspension together with a carrier, followed by drying by a conventional method, such as spray drying or lyophilising.

On producing triturate, powder, parvules, granules, tablets or the like, it is possible to use various additives, such as excipient, e.g. lactose, glucose, sucrose, and mannitol, disintegrator, e.g. starch and sodium alginate, lubricant, e.g. magnesium stearate and talc, binder, e.g. polyvinyl alcohol, hydroxypropyl cellulose and gelatine, surfactant, e.g. fatty ester, plasticizer, e.g. glycerine, and so on.

To the pharmaceutical composition, a further antioxidant may be added. The further antioxidant is not particularly limited, and may be any material having antioxidant action. Illustrative of the further antioxidants are vitamin A, such as retinal and 3,4-di-dehydroretinol, vitamin B, vitamin C, such as D-ascorbic acid and L-ascorbic acid, vitamin E, such as α-tocopherol, β-tocopherol, γ-tocopherol, δ-tocopherol, vitamin E acetate and vitamin E succinate, vitamin E phosphates, coenzyme Q, flavonoid, tannin, ellagic acid, polyphenols, radical inhibitor, hydroperoxide decomposer, metal chelating agent, active oxygen remover, carotenes, such as α-carotene, β-carotene, γ-carotene and δ-carotene, tocoquinone, and their pharmaceutically acceptable salts, and mixtures thereof.

Injections can be produced in a conventional manner where additive(s) may be added, such as pH adjustor, buffer, resolvent, suspensing agent, isotonizing agent, stabilizer, antiseptic, etc.. The injections may be lyophile preparations which can be produced in a conventional manner.

Illustrative of the suspensing agents are polysolbate 80, methyl cellulose, hydroxyethyl cellulose, sodium carboxymethyl cellulose, polyoxyethylene sorbitan monolaurate, gum Arabic, tragacanth powder, etc.. Illustrative of the resolvents are polysolbate 80, hydrogenated polyoxyethylene castor oil, nicotinamide, polyoxyethylene sorbitan monolaurate, macrogol, castor oil fatty ethyl ester, etc.. Illustrative of the stabilizers are sodium sulfite, sodium metalsulfite, etc.. Illustrative of the antiseptics are p-hydroxybenzoic acid methyl ester, p-hydroxybenzoic acid ethyl ester, sorbic acid, phenol, cresol, chlorocresol, etc..

The composition of the invention containing a tocotrienol compound can be used as or for auxiliary nutritive food, functional food, component of nutraceuticals and food additive in an arbitrary concentration.

### EXAMPLES

### Example 1

Strength of antioxidant action of tocotrienol and tocopherol on monolayer liposome and erythrocyte membrane was evaluated by using iron-ascorbic acid, 2, 2'-azobis (2-amidinopropane) - 2HC1 (AAPH) which is a water-soluble radical generator and lipoxigenase reaction system. As a result, although the difference in the strength of antioxidant action on monolayer liposome was not found between tocotrienol and tocopherol, the strength of antioxidant action of tocotrienol on crythrocyte membrane was much greater than that of tocopherol.

### Example 2

Human erythrocytes to be tested were prepared from blood of a healthy person by treating the blood according the method disclosed in Lipids, vol. 33(6), p589-595 (1998). Actually, blood drawn from a healthy person was put in a test tube containing EDTA-2Na (1mg/ml), and centrifuged at 1000×g at 4°C for 20 minutes to be separated into crythrocytes and plasma. The erythrocytes were washed three times with 5 parts by volume of phosphate-buffered saline (PBS), and centrifuged exactly at 1000×g for 20 minutes to obtain a definite volume of packed erythrocytes.

The erythrocytes were subjected to hemolysis test according to the method of Miki et al., Arch. Biochem. Biophys. 258, p373-380. 0.5 ml of the human erythrocytes were suspended into 4 parts by volume of PBS, and α-tocotrienol ethanol solution was added thereto wherein the final ethanol concentration was made 0.5%(v/v) or less. After incubating the mixture ate 37°C for 30 minutes, the erythrocytes were washed. 10% suspension of the erythrocytes was incubated again. To the suspension, 75mM AAPH-PBS solution was added, and oxidation was carried out at 37°C in a dark place with stirring. The state of erythrocyte membrane, which was destroyed by radicals to induce hemolysis, was observed.

Similarly, the human erythrocytes were incubated with α-tocopherol for 30 minutes, washed, and 10% suspension of the erythrocytes was incubated again. To the suspension, 75 mM AAPH was added, and the state of crythrocyte membrane, which was destroyed by radicals to induce hemolysis, was observed.

Reactivity of α-tocotrienol and α-tocopherol with radicals on human erythrocyte membrane was examined, and residual rate of α-tocotrienol and α-tocopherol in solution was measured after 1, 2 and 3 hours.

The results were shown below.

| | Residual Rate (%) | | |
|---|---|---|---|
| | 1hr. | 2hrs. | 3hrs. |
| α-tocotrienol | 12±2 | 7±1 | 4±1 |
| α-tocopherol | 41±3 | 25±4 | 19±3 |

From the above results, it can be seen that the decrease of tocotrienol was greater than that of tocopherol, and the incorporation (reaction rate with radicals) of tocotrienol was about three to four times as much as that of tocopherol.

The difference of the incorporation is due to the difference of structure between tocotrienol and tocopherol. Both tocotrienol and tocopherol are 8-methylcumarone-6-ol substituted with methyl group at 1-position, wherein tocopherol has a saturated chain in polyisoprene type having 16 carbon atoms. On the other hand, tocotrienol has three unsaturated chains having 16 carbon atoms, and α-, β-, γ-, and δ-isomers are different in the number and position of methyl group(s) at 5-position and 7-position.

### Example 3

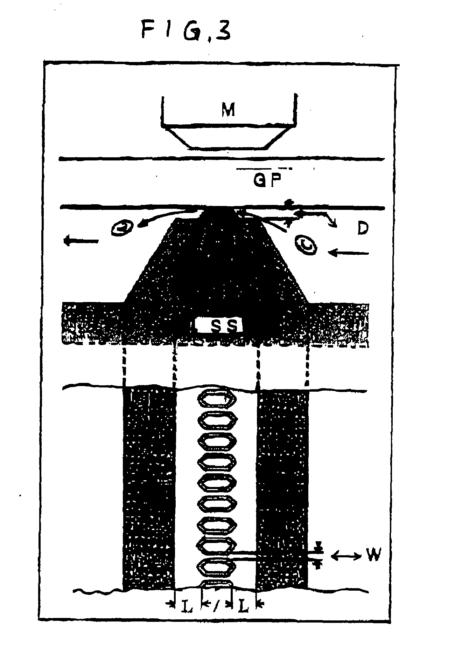

Deformability of erythrocytes was measured using a Micro-Channel Array Flow Analyzer (MC-FAN) illustrated in Figure 3 which measures time for passing total volume of an erythrocyte (8-10µ m) through a pore (6µ m) of a microchip.

Human erythrocytes were incubated with 43.3µ M α-tocotrienol for 30 minutes to incorporate the α-tocotrienol into the erythrocytes, and deformability of the erythrocytes was measured by the MC-FAN. As a control sample, human erythrocytes without α-tocotrienol were prepared, and the deformability was also measured.

As a result, passage time of the erythrocyte with α-tocotrienol was 26sec/100 µl erythrocyte (RBC), and that without α-tocotrienol was 54sec/100 µl RBC.

In comparison, human erythrocytes were incubated with 40 µM α-tocopherol (vitamin E, Tokyo Kasei Kogyo) for 30 minutes to incorporate the α-tocopherol into erythrocytes, and deformability of the erythrocytes was measured by the MC-FAN. As a result, passage time of the erythrocyte with α-tocopherol was 36sec/100 µl RBC.

The results are summarized in Table 1.

**Table 1**

| | Incorporated Compound | Passage Time (sec/100µlBC) | Ratio to Control |
|---|---|---|---|
| Example 1 | α-tocotrienol | 26±5 | 0.47 |
| Comparative | α-tocopherol | 34±5 | 0.63 |
| Control | None | 54±3 | 1.00 |

As can be seen from Table 1, tocotrienol as well as tocopherol have an action to improve deformability of erythrocytes, and the ability of improving the deformability of tocotrienol is more excellent than tocopherol. Since the tocotrienol compound has excellent ability of improving deformability of erythrocyte membrane, the pharmaceutical composition containing the tocotrienol compound is useful as an agent for improving peripheral circulation, because erythrocytes of which deformability has been improved by the tocotrienol compound can pass through blood vessel even narrowed by the deposition of cholesterol or the like.

### Example 4

Tablets having the following composition for oral administration were prepared according to a conventional method.

| | |
|---|---|
| α-tocotrienol | 40mg |
| Lactose | 40mg |
| Starch | 19mg |
| Magnesium stearate | 1mg |

### Example 5

Injections having the following composition were prepared in a form of suspension according to a conventional method.

| | |
|---|---|
| α-tocotrienol | 45mg |
| Tween 80 | 4mg |
| Puricfid water | 1mg |
| Propylparaben | 0.2mg |

### Example 6

Action of tocotrienol on the flowability of whole blood was investigated by examining 10 male persons.

42 mg/day of tocotrienol consisting of α-, γ-, and δ-isomers at a ratio by weight of 11.6 : 22.4 : 6.4 was administrated every day after dinner for 2 weeks. Blood was drawn from every person by a vacuum blood collecting tube with heparin before the investigation and after 2 weeks from the beginning of the administration each at 10 o'clock a.m.

The flowability of whole blood was evaluated by measuring passage time of 100 µl whole blood using the MC-FAN. As a result, the passage time before the ivestigation was 45.94±4.76 sec/100µ1, and that after 2 weeks was 41.43±4.93 sec/100µ1. The significant difference was less than 1%. The flowability of whole blood was improved in all examined persons.

## Claims

1. An agent for ischemic disease which comprises a tocotrienol compound as an active component.

2. An agent for improving deformability of erythrocyte membrane which comprises a tocotrienol compound as an active component.

3. An agent for improving peripheral circulation which comprises a tocotrienol compound as an active component.

4. The agent of claim 1, 2 or 3 wherein the tocotrienol compound is α-tocotrienol.
